(19) **Europäisches Patentamt / European Patent Office / Office européen des brevets**

(11) **EP 4 528 634 B1**

(12) **EUROPEAN PATENT SPECIFICATION**

(45) Date of publication and mention
of the grant of the patent:
**29.04.2026 Bulletin 2026/18**

(21) Application number: **23198720.7**

(22) Date of filing: **21.09.2023**

(51) International Patent Classification (IPC):
*G06T 7/00* (2017.01)      *G06T 7/90* (2017.01)
*G06V 10/25* (2022.01)      *G06V 20/69* (2022.01)
*A61B 5/00* (2006.01)

(52) Cooperative Patent Classification (CPC):
**G06T 7/0012; A61B 5/444; G06T 7/90;
G06V 10/25; G06V 20/698;** A61B 5/6898;
G06T 2207/30088; G06T 2207/30096

(54) **METHOD OF BIOIMAGE ANALYSIS FOR THE DETECTION OF COLOR ABNORMALITIES THROUGH COLOR DISTRIBUTION ASSESSMENT**

VERFAHREN ZUR BIOBILDANALYSE ZUR DETEKTION VON FARBANOMALIEN DURCH FARBVERTEILUNGSBEURTEILUNG

PROCÉDÉ D'ANALYSE D'IMAGES BIOLOGIQUES POUR LA DÉTECTION D'ANOMALIES DE COULEUR PAR ÉVALUATION DE DISTRIBUTION DE COULEUR

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(43) Date of publication of application:
**26.03.2025 Bulletin 2025/13**

(73) Proprietor: **BIOPIX DNA TECHNOLOGY S.A.
70013 Heraklion, Crete (GR)**

(72) Inventors:
 • **XHIXHO, Jorgo**
  GR-71202 Heraklion, Crete (GR)
 • **KOUROUGKIAOURI, Despoina**
  GR-71307 Heraklion, Crete (GR)
 • **CHATZIIOANNIDOU, Styliani**
  GR-71202 Heraklion, Crete (GR)
 • **PAPADAKIS, George**
  GR-71305 Heraklion, Crete (GR)

(74) Representative: **Dodou, Evdokia
Katsimpiri 3
15561 Cholargos, Athens (GR)**

(56) References cited:
 • **T-T DO ET AL: "Accessible Melanoma Detection using Smartphones and Mobile Image Analysis", ARXIV.ORG, CORNELL UNIVERSITY LIBRARY, 201 OLIN LIBRARY CORNELL UNIVERSITY ITHACA, NY 14853, 27 November 2017 (2017-11-27), XP080840297**
 • **MAHDIRAJI SAEID AMOUZAD ET AL: "Skin lesion images classification using new color pigmented boundary descriptors", 2017 3RD INTERNATIONAL CONFERENCE ON PATTERN RECOGNITION AND IMAGE ANALYSIS (IPRIA), IEEE, 19 April 2017 (2017-04-19), pages 102 - 107, XP033125833, [retrieved on 20170717], DOI: 10.1109/PRIA.2017.7983026**
 • **ALI ABDER-RAHMAN H ET AL: "Automating the ABCD Rule for Melanoma Detection: A Survey", IEEE ACCESS, IEEE, USA, vol. 8, 28 April 2020 (2020-04-28), pages 83333 - 83346, XP011787841, [retrieved on 20200512], DOI: 10.1109/ ACCESS.2020.2991034**

EP 4 528 634 B1

**Description**

TECHNICAL FIELD

The present invention relates to biomedical image analysis methods for the detection of a lesion, and specifically for the detection of melanomas, in a digital image of a tissue. It also relates to apparatuses adapted to perform said methods as well as computer programs for use in said methods.

BACKGROUND OF THE INVENTION

Melanoma is an aggressive form of skin cancer with rapidly rising incidence. In the early stages, melanoma can be treated successfully with surgery alone and survival rates are high, but after metastasis survival rates drop significantly. Therefore, early and correct diagnosis is important for ensuring patients have the best possible prognosis. Melanoma misdiagnosis accounts for more pathology and dermatology malpractice claims than any cancer other than breast cancer, as malignant melanoma can disguise clinically as benign lesions (false negatives), and benign pigmented lesions can clinically simulate malignant melanoma (false positives), thus there is a clear need for tools that aid clinicians and improve workflow.

Digital microscopy has become an essential tool in pathological research over the past few decades. The generation of digital images of histological tissue has allowed the creation and application of image analysis algorithms. Image analysis can be defined as the combination of computational techniques for the automatic processing of carefully captured images to produce datasets of measurements and extract useful information. The images themselves can come from a variety of sources, from digital cameras or smartphones to more advanced sensors integrated into sophisticated instruments. Image analysis has already been proposed to be useful for extracting biologically relevant information in applications such as detection of plant diseases, determination of food quality and safety, diagnosis of skin cancer and many more. In all such cases, the purpose of analyzing images (bioimage analysis) is to spot abnormalities that can be attributed to a biological phenomenon when compared to a known (normal) condition.

Bioimage analysis can be achieved with excellent outcomes by employing dedicated instrumentation with specific camera sensor features. However, in the current era of smartphone diagnostics, building dedicated instrumentation is considered outdated and in addition cannot satisfy the need for rapid, simple, and inexpensive point-of need diagnostics or self-testing. Smartphone applications performing bioimage analysis for diagnostic purposes lack in accuracy because of a) differences in smartphone cameras specifications, b) variation in lighting conditions during image capturing and c) photos that are taken by non-experts. As an alternative, the deep-learning technology, known as the AI method, has become a new trend since it is not affected by the aforementioned problems. However, AI bioimage analysis requires high quality photos that are pre-processed manually, which can be time-consuming and can vary, depending on the skill of the person. In addition, prior art bioimage analysis is challenged by insufficient training data, interpretability, and requirements for high computational power. Most importantly, prior art methods of diagnostic bioimage analysis based on the detection of color abnormalities do not provide suitable metrics for the quantitative assessment of the likelihood that a skin lesion is melanoma.

Do T-T et al. (IEEE Transactions on Multimedia, 2018, 20(10):2849 - 2864) discloses an image analysis method for the detection of melanoma, which employs a Lesion Color Feature, which calculates the color features (mean, variance) of the pixel values of several color channels, and a Color Triangle feature which helps determine if there is color variation from the lesion center to the border.

Mahdiraji SA et al (2017 3rd International Conference on Pattern Recognition and Image Analysis (IPRIA), 2017, pages 102-107) proposes a method for skin lesion images diagnosis which employs feature sets that describe the color variation at the lesion boundary.

Abder-Rahman HA et al (IEEE Access 2020, 8:83333 - 83346) reviews the different methods proposed in literature towards automating the ABCD rule ((Asymmetry, Border irregularity, Color variegation, Diameter) for detection of melanoma.

Therefore, the need remains for image analysis methods that provide an easy to implement, highly accurate procedure for the discrimination of malignant melanoma from benign skin lesions.

SUMMARY OF THE INVENTION

The invention described herein provides a method according to claim 1 that solves the problems of the prior art. Specifically, the present invention provides a computer implemented method of detecting melanoma in a digital skin image, comprising the following steps:

a) determining the area of interest (AOI) in the digital image through an image segmentation algorithm, masking the AOI from the outside area, and converting the masked AOI and the area outside the AOI into grayscale;

b) calculating the mean value and the standard deviation for the AOI and for the area outside the AOI to extract a risk threshold as a function of the mean and standard deviation;

c) dividing the color of the AOI into contours by applying a grayscale intensity threshold value to the image;

d) if multiple contours are identified in step (c), measuring the mass center of all contours identified and calculating the euclidean distance of each contour center from said mass center of all contours;

e) for each contour identified in step (c), calculating the ratio of the average plus the standard deviation of all the measured distances divided by each contour's distance from the mass center of all contours measured in step (d);

f) comparing said ratio with the risk threshold extracted in step (b) whereby, if said ratio is smaller than the risk threshold extracted in step (b), then said contour is classified as having abnormal color distribution; and

g) repeating steps (c) to (f) by applying multiple threshold values to the image, until the size of a contour covers the AOI;

wherein the presence of multiple contours having abnormal color distribution and whose numbers are increasing during the process is an indication of a melanoma.

The method described herein facilitates the detection of color abnormalities during bioimage analysis to characterize a particular skin condition as "normal" or "not normal" when compared to a known physiological state, and specifically for the discrimination of malignant melanoma from benign nevi. The method can also be adapted for use in a range of medical, biological, or other applications such as the detection of plant diseases or food quality deterioration. It is not based on AI and does not depend on the camera sensor specifications and the lighting conditions during picture capturing. It can also be integrated into smartphone applications for usage by non-expert individuals and with low computation power.

The method assesses the color distribution in a particular image area of interest by employing a series of mathematical calculations that must be performed in a particular order. These calculations result in the identification of color contours and the relative distances among them within the area of interest. The advantage for the disclosed method compared to prior art image analysis methods is that it provides a quantitative method for assessing the likelihood that a particular skin lesion is a melanoma or a benign nevus. As will be shown in the attached Examples, the method of the present invention discriminates malignant melanoma with high accuracy that reaches or exceeds 93%, which is greater than the accuracy normally achieved by the trained eye of a medical expert which rarely exceeds 70%.

The invention also provides an apparatus for detecting a melanoma in a digital image of a skin tissue, the apparatus comprising a camera adapted to obtain said image, a processor adapted to process the image data according to the method disclosed herein, and an output device adapted to indicate a likelihood of the presence or absence of a melanoma.

Also provided is a computer program comprising instructions which, when the program is executed by a computer, cause the computer to carry out the method disclosed herein.

Other aspects and benefits of the present invention will become apparent from the detailed description to follow.

BRIEF DESCRIPTION OF THE DRAWINGS

The present invention will now be described with reference to certain embodiments thereof which are illustrated in the accompanying drawings. It should be noted that the accompanying drawings illustrate preferred embodiments of the invention, therefore should not be considered as limiting the scope of the invention.

Fig. 1 shows a schematic flowchart diagram of a method of detecting an abnormal morphological feature in a digital image of a skin tissue according to the invention.

Fig. 2 shows the workflow of a method of detecting an abnormal morphological feature in a digital image of a skin tissue according to a preferred embodiment of the invention.

Fig. 3 shows exemplary output graphs according to the invention. An increase in the number of contours classified as having abnormal color distribution ("risky contours") during the process (repetitions) as depicted in the top curves (A) is an indication of the presence of a melanoma. A steady or decreasing number of contours having abnormal color distribution (B) corresponds to a benign skin condition.

Fig. 4 is a presentation of the contour process applied to a melanotic nevus (A) and a melanoma (B) through a series of images. In the nevus the color is expanding uniformly, while in the melanoma it expands in a random way.

Fig. 5 is a diagrammatic presentation of the process of calculating the number of peaks around the borders of the AOI. (A) corresponds to a digital image of a melanoma and (B) corresponds to a digital image of a nevus.

Fig. 6 is a diagrammatic presentation of the process of calculating the optimal $R_c$. (A), (C), (E) and (G) represent the results from the training set; (B), (D), (F) and (H) represent the results from the test set. The $R_c$ values tested were 0.25 (A, B), 0.24 (C, D), 0.22 (E, F) and 0.22 (G, H). The result obtained at the end of this process was $R_c$ = 0.232 (I).

Fig. 7 shows dermoscopic images of a nevus (A) and a melanoma (C) and the corresponding output for the nevus (B) and for the melanoma (D).

DETAILED DESCRIPTION OF THE INVENTION

The present disclosure provides a computer implemented image analysis method for diagnosing melanoma in a digital skin image. It also provides an apparatus for detecting a melanoma in a digital image of a tissue as well as a computer program for implementing said method.

The term "contour centroid" or "centroid" as used throughout the description and claims refers to the geometric center of a contour.

The term "sub-contour" as used throughout the description and claims refers to any contour that is detected inside a contour.

The method refers to the assessment of color distribution in an image which can be used to characterize a particular skin condition as "normal" or "not normal" when compared to a known physiological state. The image can be taken by a digital camera, a smartphone camera, or any other kind of sensing elements integrated into an instrument. The abnormal or non-physiological state is a condition that does not appear naturally but instead it can be attributed to a biological phenomenon such as the growth of cancer cells.

The invention discloses an iterative process for quantitatively analyzing a digital image of a melanotic and non-melanotic skin lesion for the detection of color distribution abnormalities. Specifically, the inventors have found that if the number of contours classified as having abnormal color distribution increases during said process, this is an indication of the presence of a melanoma. A steady or decreasing number of contours classified as having abnormal color distribution corresponds to a benign skin condition such as a nevus.

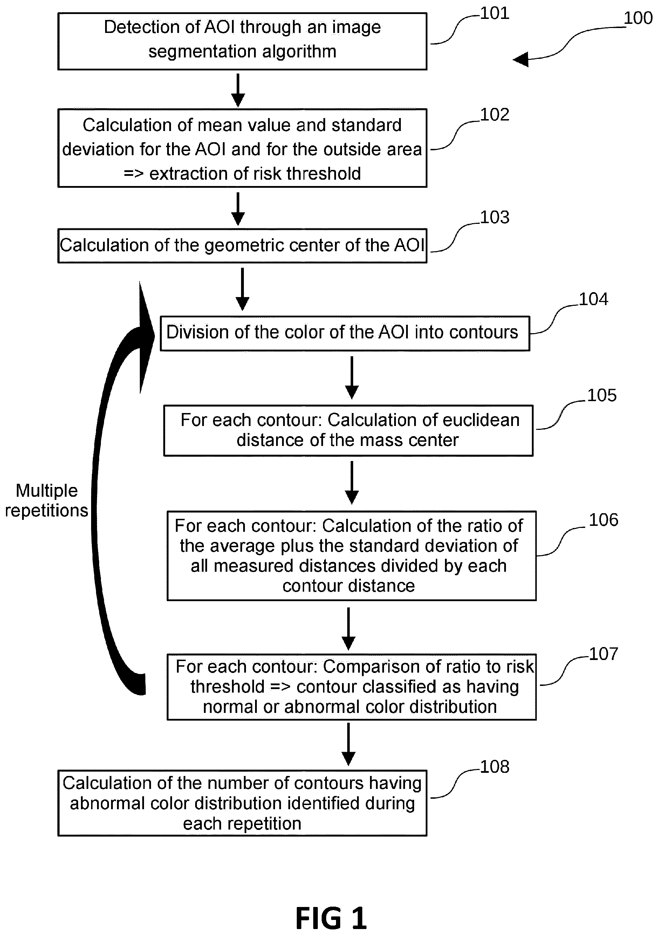

The detection of color abnormalities in a particular image is achieved by following a series of distinct mathematical functions (Figure 1). In step 101, the area of interest (AOI) which corresponds to the part of the image that is checked for color abnormalities is first defined using an image segmentation algorithm. The AOI is thereby separated from the outside area (OA). This step is important for color normalization purposes. Subsequently, the AOI and the OA are converted into grayscale.

In step 102, the mean color intensity value and the standard deviation for the AOI and for the area outside the AOI are calculated. This step results in the calculation of a risk threshold as a function of the mean and standard deviation. The risk threshold is used in step 107 to determine whether each of the contours identified has normal or abnormal color distribution.

In step 103, the method then calculates the geometric center of the AOI. Since the shape of interest is only one, the centroid and the mass center are identical. In a preferred embodiment, the method also calculates the symmetry of the AOI. If the AOI is asymmetrical, this is an indication of a malignant lesion. In another preferred embodiment, the noise at the borders of the AOI and specifically the peaks around the borders are measured. This is achieved, first by measuring the distance of each pixel at the border of the area of interest and subsequently calculating the points that intersect the mean value and the distance. An anomalous border of the AOI is an indication of a melanoma. In a more preferred embodiment, the method calculates both the symmetry of the AOI and the noise at the borders of the AOI.

In step 104, the color of the AOI is divided into contours. During this step, a threshold value is applied to the image and said intensity threshold value creates one or more contours. In a preferred embodiment, threshold values start from the first intensity level that the global threshold detects.

In step 105, if more than one contours are identified in the previous step, the euclidean distance of the mass center of each contour identified is calculated and in step 106, for each contour identified a ratio is calculated of the average plus the standard deviation of all the measured distances divided by each contour distance.

In step 107 the resulting ratio is compared to the risk threshold calculated in step 102, whereby each identified contour is classified as having normal or abnormal color distribution.

In a preferred embodiment, the method additionally comprise a subsequent step wherein, if a sub-contour is detected inside a contour, the average color intensity of an area around the center of the sub-contour and the average color intensity of an area around the center of the corresponding main contour are measured to determine if the intensity of the sub-contour is significantly different from that of the corresponding main contour. Preferably, the area around the center of the sub-contour and the corresponding main contour whose average color intensity is measured should extend from 5 or more pixels towards all directions. More preferably, the area around the center of the sub-contour and the corresponding main contour whose average color intensity is measured should extend from 5 to 15 pixels towards all directions. A sub-contour whose intensity is significantly different from that of the main contour is classified as having abnormal color distribution.

Steps 104, 105, 106 and 107 are repeated until the size of a contour covers the AOI. During each repetition of step 104, a different threshold value is applied, by increasing the value so that each time the contour covers a larger area.

In step 108, the number of contours (and optionally sub-contours) having abnormal color distribution identified during each repetition is calculated. As will be shown in the Examples, the presence of multiple contours having abnormal color distribution whose numbers are increasing during the process highly correlates with the presence of a melanoma.

The method disclosed herein is used to determine the possibility that a skin lesion is a melanoma. In view of the serious nature of melanoma, and the extreme importance of early detection of the disease, a method is needed to allow individuals who may be concerned that they have melanoma to obtain a rapid, preliminary screening using a computer-based image analysis and pattern recognition tool that is easily accessible, for instance via the Internet, and which can utilize readily-available imaging techniques such as a smartphone camera or convention digital camera. The present invention expands the methods disclosed in the prior art to provide such a capability.

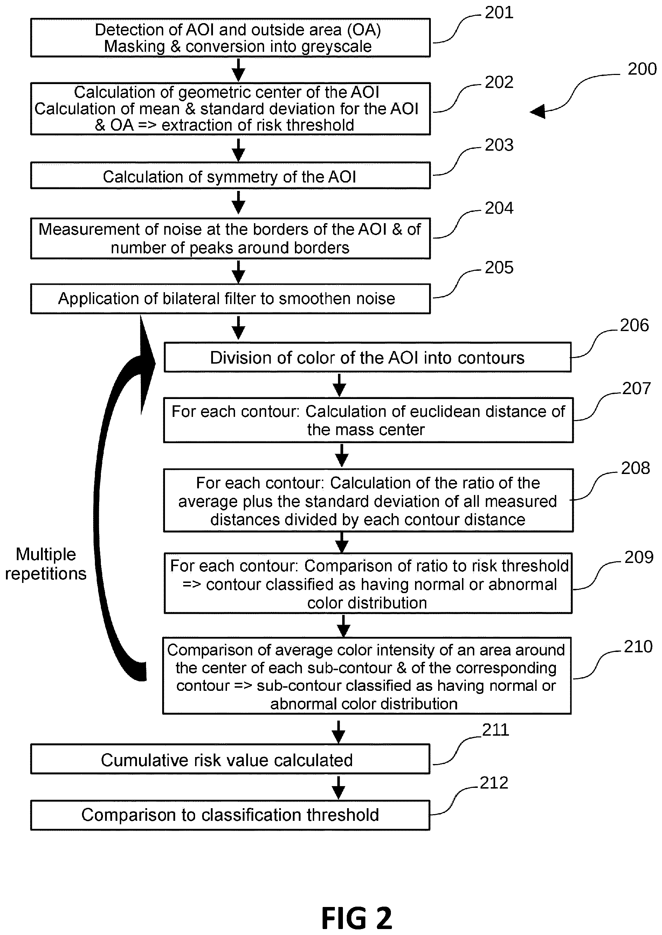

Figure 2 is a flow diagram illustrating a preferred embodiment of the computer implemented method 200 of detecting a melanoma in a digital image of a tissue. The method 200 is exemplary. In use, the method 200 may include additional steps, omit some steps, and/or perform certain steps in different order. The method 200 is presented as an overview. Many of the specifics of performing the various steps of the method 200 are described in detail herein.

In step 201, a skin image, such as a dermoscopic image or an image taken by a smartphone camera, examined for the presence of lesions, is divided into two parts, the one part is the AOI, and the second part is the OA (outside area). The area of interest gets unified as one and masked from the outside part (masked image). Subsequently, the two areas are converted into grayscale.

In step 202, two vectors are generated, one that corresponds to OA and the other to the AOI. The mean color intensity and standard deviation of both areas is then calculated, preferably by random picking from 60% to 70% of the corresponding vector. A risk threshold is then calculated as a function of the mean and standard deviation calculated during this step. When the standard deviation is high, there is high probability of unwanted noise, such as blurry image, presence of hair, freckles etc.). Subsequently, the geometric center, or centroid, of the AOI and the OA is calculated as a function of pixel intensity.

In step 203, the symmetry of the AOI is calculated. To define the symmetry of a lesion, an ellipse is fitted on the masked area. If the area is symmetrical, then the area of the ellipse and the masked area should converge at about 90% or higher, preferably at 90% plus or minus 2% or higher. If the AOI is asymmetrical, it is assigned a risk value indicating an increased probability of the presence of a malignant lesion.

In step 204, the noise at the borders of the AOI and specifically the peaks around the borders are measured. This is achieved, first by measuring the distance of each pixel at the border of the area of interest and subsequently calculating the points that intersect the mean value and the distance. If are more than 5 intersected points are calculated, the AOI has anomalous borders and it is assigned a risk value indicating an increased possibility of the presence of a lesion.

In step 205, a bilateral filter is applied to smoothen the noise or unwanted detail. The filter applied, which is a function of the standard deviation for the AOI, depends on the resolution of the image which is analyzed. For instance, when a dermoscopic image is analyzed, the recommended values tend to be higher, whereas when phone images, with high resolution camera, are taken to be analyzed, the recommended values tend to be lower.

In step 206, the color of the AOI is divided into contours, preferably by using binary (global) thresholding. During this step, a threshold value is applied to the grayscale image and this grayscale intensity threshold value creates one or more contours. Threshold values start from the first intensity level that the global threshold detects.

In step 207, if more than one contours are identified in the previous step, the euclidean distance of the mass center of each contour identified is calculated and in step 208, for each contour identified, a ratio is calculated of the average plus the standard deviation of all the measured distances divided by each contour distance. In step 209, the resulting ratio is compared to the risk threshold calculated in step 202, whereby each identified contour is classified as having normal or abnormal color distribution.

In step 210, if a sub-contour is detected inside a contour, the average color intensity of an area around the center of the sub-contour and the average color intensity of an area around the center of the corresponding main contour are measured to determine if the intensity of the sub-contour is significantly different from that of the corresponding main contour. Preferably, the area around the center of the sub-contour and the corresponding main contour whose average color intensity is measured should extend from 5 or more pixels towards all directions. More preferably, the area around the center of the sub-contour and the corresponding main contour whose average color intensity is measured should extend from 5 to 15 pixels towards all directions. A sub-contour whose intensity is significantly different from that of the main contour is classified as having abnormal color distribution. As an example, the average color intensity of the sub-contour is considered as significantly different from that of the corresponding main contour if it corresponds to a different basic color to that of the corresponding main contour. If the image is in grayscale, the average color intensity of the sub-contour is considered as significantly different from that of the corresponding main contour if it corresponds to a different basic color in the grayscale range. For instance, if the difference of the mean values of the two areas (sub-contour and contour) is higher than 30 the average color intensity of the sub-contour is considered as significantly different from that of the corresponding main contour. The value of 30 emerges considering that there are 8 basic colors (white, black plus the six basic colors produced by the three main colors). The grayscale values extend from 0 to 255, so 255 divided by 8 equals 31.2, such that the white shades correspond to values of 0 to 30, yellow shades correspond to values of 30 - 60 etc. If the average color intensity of the sub-contour and contour is, for example, 150 and 100 respectively, they belong to different "packs" of color shades.

Subsequently, steps 206, 207, 208, 209 and 210 are repeated until the size of a contour covers the AOI. Preferably, steps 206, 207, 208, 209 and 210 are repeated until the size of a contour reaches the 75% to 80% percent of the lesion area (AOI). The 75% to 80% values correspond to the optimum percentage as it covers the maximum AOI excluding the unwanted information at the borders, such as shadows and dark spots, which might be caused by imperfect segmentation. As each skin lesion has different shades of colors which are distributed differently, the contours also converge differently. Thus, the range of the threshold levels depends on the object that is depicted in the image, and for that reason it is not always the same. During each repetition of step 206, a different threshold value is applied, by increasing the value so that the contour increasingly covers a larger area. Preferably the intensity threshold value applied during each repetition of step 206 increases by a value of one.

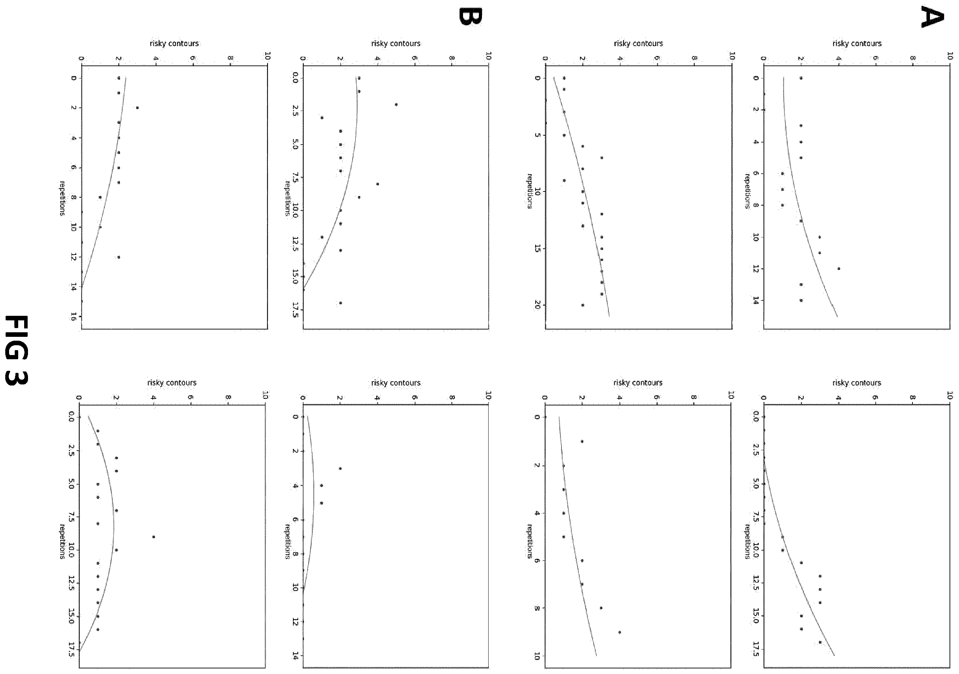

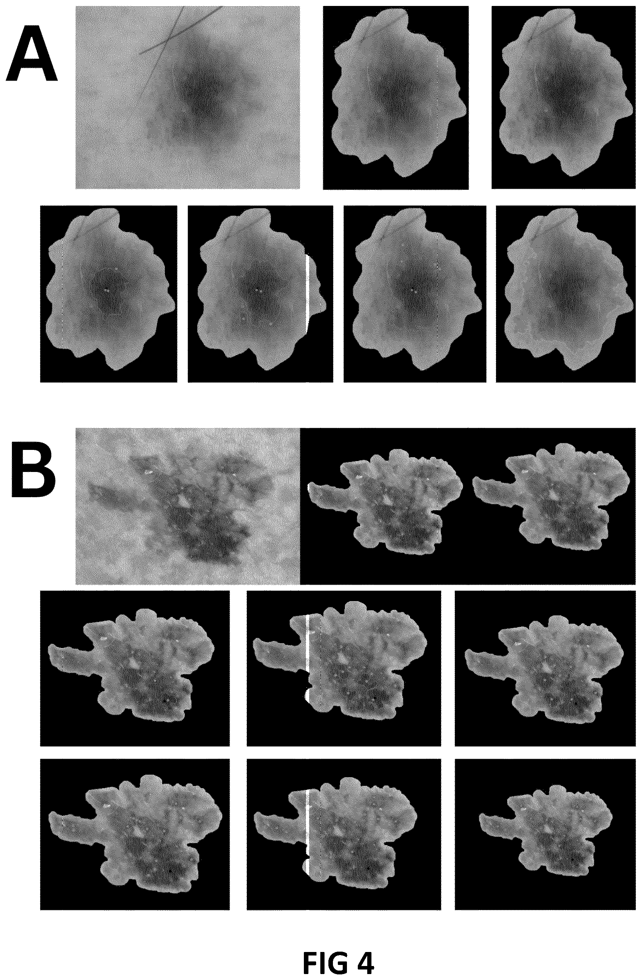

The evaluation outcome of the above steps is used to determine whether the color distribution indicates the existence of melanoma. More specifically, in Figure 3, the difference between the distribution of contours with abnormal color distribution during the iterations of the described process, detected in melanotic and non-melanotic lesions is presented. As the algorithm proceeds from one repetition to the other and detects the contours, the ones classified as having abnormal color distribution ("risky contours") are plotted against the number of repetitions taking place each time throughout the process. In case of a melanoma, an increasing curve is created as the number of repetitions increases, indicating the abnormal color distribution observed in such lesions (Figure 3A). When the lesion is non-melanotic, the curve shown is steady or decreasing (Figure 3B), depicting the relatively even color distribution seen to a non-melanotic compared to a melanotic lesion. Similarly, Figure 4 shows through a series of images that, when the contour process is applied to a melanotic nevi (Figure 4A) the color is expanding uniformly, whereas when it is applied to a melanoma (Figure 4B), the color expands in a random way.

During each repetition of steps 206 to 210, a risk value may be calculated in order to provide a quantitative assessment of the growth rate at which the number of contours with abnormal color distribution increases during the iterative process. Said risk value increases every time a contour or sub-contour is classified as having abnormal color distribution. For instance, one unit is added to the risk value every time a contour or sub-contour is classified as having abnormal color distribution. A cumulative risk value is subsequently calculated (step 211) at the end of the iterative process, which provides a quantitative assessment of the probability of the presence of a melanotic lesion. In another preferred embodiment, the symmetry of the AOI and/or the noise at the borders of the AOI are also taken into consideration when calculating the cumulative risk value. For instance, two units may be extracted from the cumulative risk value if the AOI is both symmetrical and with smooth borders; two units may be added to the cumulative risk value if the AOI is asymmetrical and has anomalous borders; one unit may be extracted from the cumulative risk value if the AOI is asymmetrical with smooth borders or symmetrical with anomalous borders.

If a cumulative risk value is calculated in step 211, the ratio of the cumulative risk value divided by the total number of repetitions is compared to a classification threshold in step 212. The inventors have analyzed a large number of medical images from melanoma and non-melanoma cases (Example 1) and have concluded that the classification threshold $R_c$ ranges from 0.18 to 0.25, (0.18, 0.25). The exact value depends on the image, i.e., whether it is a dermoscopic image or a smartphone image. The person skilled in the art looking to determine the exact value of the classification threshold $R_c$ for the particular images used, would use a few known samples (melanotic and non-melanotic) to calibrate the algorithm, as explained in Example 1.

If the ratio of the cumulative risk value divided by the total number of repetitions is smaller than the classification threshold $R_o$, the lesion is non-melanotic, whereas if the ratio is higher than the $R_e$, the lesion is melanotic.

EXAMPLES

Example 1

Calibration of the methodology - Calculation of the $R_c$ value

The method disclosed herein was used to analyze 44 medical images containing 15 known melanoma and 29 non-melanoma (nevi) cases (Figure 4). The images are randomly picked to avoid bias in the results. The methodological steps were incorporated into a computer algorithm which analyzed every image independently.

[1] Each image under analysis was divided into two parts, the one part being the area of interest, and the second part being the outside part (surrounding area) by applying the KNN algorithm. The area of interest was subsequently unified as one and masked from the outside part (masked image).

[2] The two areas were converted into grayscale. For that reason, the pixels of each area were read as a one-dimensional vector. For the conversion the luminance method was used, $vector_{image}$ = 0.299 ·Red+0.587 · Green+0.114 ·Blue. Also, the same method was used to convert the masked image into a grayscale.

[3] From step [2], two vectors were generated, one corresponding to the outside area and the other to the area of interest, (OA, AOI). The mean value and standard deviation is calculated for each vector by random picking from 60% to 70% of it, $(E_{OA}, \sigma_{OA})$, $(E_{AOI}, G_{AOI})$. The definition of these values facilitates the determination of the condition and the intensity texture of skin (OA) and the AOI. An increased $\sigma$ value corresponds to more "noisy" texture. More specifically, a "noisy" texture is defined by a non-uniform color distribution, meaning that the skin has many small differentiation points (e.g., freckles) and these skin areas are in a higher risk to develop skin cancer originating from moles. Also, lower values of $E_{AOI}$ means darker colors.

[4] The centroid, which is the center of the shape/ surface of the area of interest (initial centroid) was defined by using the "image moment" equation for the masked part.

$$Centroid = (\bar{x}, \bar{y}) = \left( \frac{M_{1,0}}{M_{1,0}}, \frac{M_{0,1}}{M_{0,0}} \right)$$

$$M_{i,j} = \sum_x \sum_y (x - E(x))^i (y - E(y))^j I(x, y)$$

$$M_{0,0} = \sum_x \sum_y I(x, y)$$

where I(x, y) is the pixel intensity of the corresponding (x, y) coordinates.

[5] The shape of a nevus, or benign lesion, tends to be rounder and is classified as symmetrical. This can also be seen in Figure 4, where benign skin lesions (Figure 4A) have a rounder shape compared to melanoma (Figure 4B). To define the symmetry of a lesion, an ellipse was fitted on the masked area. If the area is symmetrical, then the area of the ellipse and the masked area should converge $\geq$ 90%. The elliptical area is calculated from $El_{area} = \pi \cdot A \cdot B$, where A is the length of the semi major axis and B the length of the semi minor axis, and convergence is calculated as follows:

$$S = \frac{|El_{area} - Mask_{area}|}{Mask_{area}}$$

$$\begin{cases} symmetrical, S < 10\% \\ asymmetrical, S \geq 10\% \end{cases}$$

[6] The noise at the borders of the area of interest and specifically the peaks around the borders were measured. Every pixel on the border of a lesion can be considered as a (x, y) 2-dimensional space vector. The euclidean distance of each $(x_i, y_i)$ point from its lesion center $(c_x, c_y)$ was calculated using the formula:

$$d_i = \sqrt{(c_x - x_i)^2 + (c_y - y_i)^2}$$

Next, all calculated distances were normalized by subtracting the average value of them. Let $\hat{d}$ be each normalized result. If the lesion is a perfect shape, we expect $\hat{d}$ to be constant and zero (the subtraction of the constant numbers from the average value of constant numbers is zero). If the lesion is elliptically shaped, it would look like a trigonometrical graph, with four peaks (two extreme and two minimum). If the geometrical shape of the lesion is not perfect, the number of peaks would be more than five. In the latter case the borders are considered abnormal ("noisy"). If $\widehat{d_i} \cdot \widehat{d_{i+1}} < 0$ this signifies the existence of a peak. The number of peaks were calculated using the formula below:

$$peaks = \sum_{i=0}^{n-1} 1_{\widehat{d_i} \cdot \widehat{d_{i+1}} \leq 0}$$

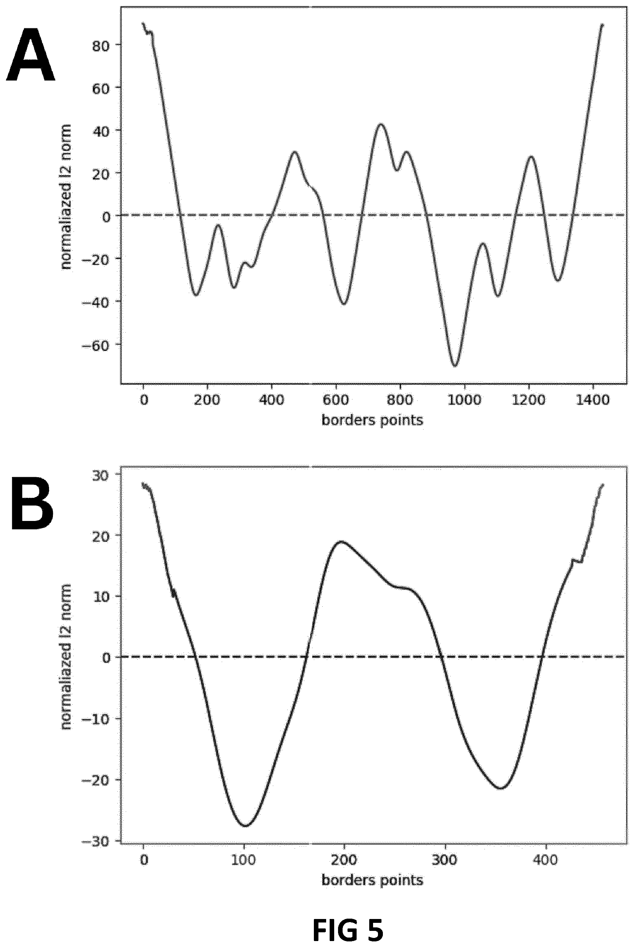

Figure 5 is a diagrammatic presentation of the process of calculating the number of peaks around the borders of the AOI. Figure 5A corresponds to a digital image of a melanoma and Figure 5B corresponds to a digital image of a nevus. [7] A Gaussian filter was applied to smoothen the noise or unwanted detail. The parameters set for $\sigma_x \in (5, 11)$ and $\sigma_y \in (5, 11)$ depend on the images which are analyzed. For example, when dermatoscopic images are analyzed, the recommended values of $\sigma_x$, and $\sigma_y$ are: $\sigma_x$, = 11, $\sigma_y$ = 11. When phone images, with high resolution camera are taken to be analyzed, the recommended values of $\sigma_x$ and $\sigma_y$ are lower, $\sigma_x$, = 7, $\sigma_y$ = 7.

[8] A risk threshold $R_{th}$ was defined, considering the results from the step [3].

$$R_{th} = 0.99 \, if \left( \sigma(O) > 25 \cap \sigma(I) \geq 17 \right)$$

$$R_{th} = 0.97 \, if \sigma(I) \geq 17$$

$$R_{th} = 0.96 \, if \sigma(O) > 25$$

$$A = \left( \sigma(I) < 17 \cap \sigma(O) < 20 \right), B = E(O) - \sigma(O) - E(I) - \sigma(I)$$

$$R_{th} = \left\{ \begin{array}{c} 0.90, A \\ 0.85, B \cap A \end{array} \right\}$$

[9] The color of the area of interest was divided into contours, starting from darker to lighter ones. To achieve this, multiple conversions of the smoothed masked image to a binary one (black and white) were performed, using binary (global) thresholding. During this procedure, multiple threshold values were applied to the grayscale image. Threshold levels started from the first intensity level that the global threshold detected, until it reached the 75% to 80% percent of the lesion area. The range of the threshold levels depends on the object that is depicted in the image, and for that reason it is not always the same.

[10] Each grayscale intensity threshold value, created one or more contours. In every case where there were more contours than one, the mass center of all the contours was measured, by using:

$$C_x = \frac{\sum_i C_{ix} A_i}{\sum A_i}, \quad C_y = \frac{\sum_i C_{iy} A_i}{\sum A_i}$$

$A_i$ is the area of each contour, and if $Area = \sum A_i$, the formula can be written:

$$C_x = \frac{\sum_i C_{ix} A_i}{Area_i}, \quad C_y = \frac{\sum_i C_{iy} A_i}{Area_i}$$

The second step of this procedure included the calculation of the euclidean distance of each contour center from the new center, C'.

$$D_i' = \sqrt{\left( C_{ix} - C_x' \right)^2 + \left( C_{iy} - C_y' \right)^2}$$

thereby creating a one dimensional array, $D = (D'_0, D'_1, D'_2, ..., D'_n)$, where n is the number of contour centers. At the end, each contour was classified as having normal or abnormal color distribution: The second step of this procedure included the calculation of the euclidean distance of each contour center with the new center, C'.

$$D_i' = \sqrt{\left(C_{ix} - C_x'\right)^2 + \left(C_{iy} - C_y'\right)^2}$$

thereby creating a one dimensional array, D = (D'$_0$ , D'$_1$ , D'$_2$ , ...,D'$_n$), where n is the number of contour centers. At the end, each contour was classified as having normal or abnormal color distribution:

$$r_i = \left\{ \begin{array}{l} 1, \dfrac{\left( E(D) + \sigma(D) \right)}{D_i'} < R_{th} \\ 0, otherwise \end{array} \right\}$$

And it returns:

$$r = \sum_i r_i$$

[11] For every sub-contour that was detected inside a contour during step [10], the area around its center was measured, to determine if it represented a significant change inside the main contour. The area measured extended from 5 to 15 pixels towards all directions, from both contour and its sub-contour area, except, if the sub-contour was located at the main contour center, then another area was chosen randomly. Any significant change at this step meant that the intensity of the two areas was completely different. If the difference of the mean values of the two areas (sub-contour and contour) was higher than 30, the average color intensity of the sub-contour was considered significantly different. If a sub-contour with a significant change had been detected, one risk value was added to the step [10] result, meaning that from this point the value r was defined as $r_{i+1} = r_i + 1$.

[12] Steps [10] and [11] were repeated until step [9] reached the desired covered area. The method for each iteration returned a risk value, $r_i$, which means that a one dimensional (1D) array $\hat{r}$ was added.

[13] Finally, the symmetry and the borders that arose from step [5] and [6] respectively, was added as extra feature, r', in the resulting 1D array $\hat{r}$ from step [12].

$$r' = \left\{ \begin{array}{l} \left( \sum_i r_i \right) - 2, (Symmetrical \cap SmoothBorders) \\ \left( \sum_i r_i \right) - 1, (Symmetrical \cup SmoothBorders) \\ \left( \sum_i r_i \right) + 2, (Asymmetrical \cap AnomalousBorders) \\ \left( \sum_i r_i \right) - 1, (Asymmetrical \cup AnomalousBorders) \end{array} \right\}$$

[14] The classification is defined by:

$$class = \left\{ \begin{array}{l} normal, \dfrac{\widehat{r}_n}{k} < R_c \\ abnormal, \dfrac{\widehat{r}_n}{k} > R_c \end{array} \right\}$$

k, is the number of irritations it took the method to end, $\widehat{r}_n$ is the last value of the array $\hat{r}$ and $R_c$ is the threshold that defines the classification.

For the calculation of $R_c$, the data were split to a training set (80%) and a test set (20%).

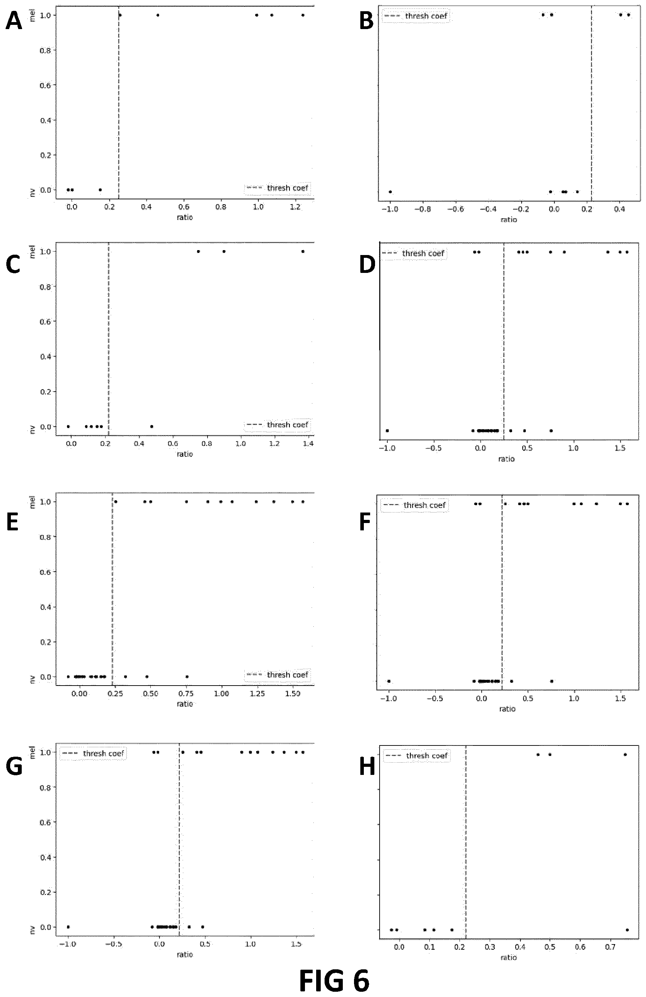

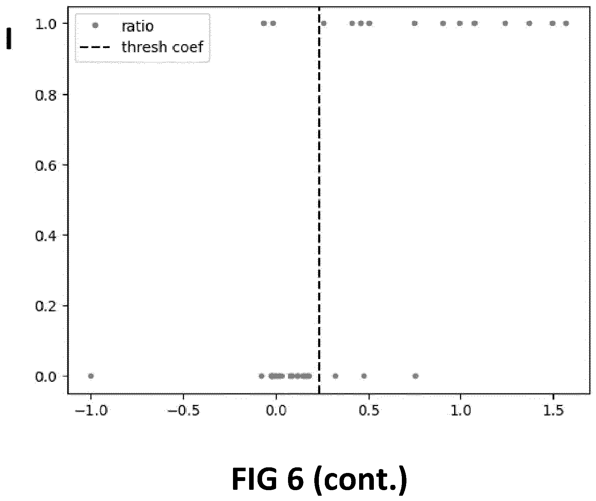

- Starting from Rc values of 0.18 to 0.25, all values were tested until a $R_c$ value was calculated that classified the most known melanoma cases as melanomas and had the lowest percentage of nevi lesions classified as melanoma. Figure 6 is a diagrammatic presentation of this process. Figures 6A, 6C, 6E and 6G represent the results from the training set; Figures 6B, 6D, 6F and 6H represent the results from the test set. The $R_c$ values tested were 0.25 (Figures 6A, 6B), 0.24 (Figures 6C, 6D), 0.22 (Figures (Figures 6E, 6F) and 0.22 (Figures 6G, 6H).

- The Rc value was evaluated with the test data and the score was recorded.

- The same process was repeated multiple times by shuffling the data.

- At the end of this process, the $R_c$ was the average score from all the trials.

The result obtained at the end of this process was $R_c$ = 0.232, (Figure 6I) scoring accuracy of 88%, sensitivity = 86% and specificity = 89.6%.

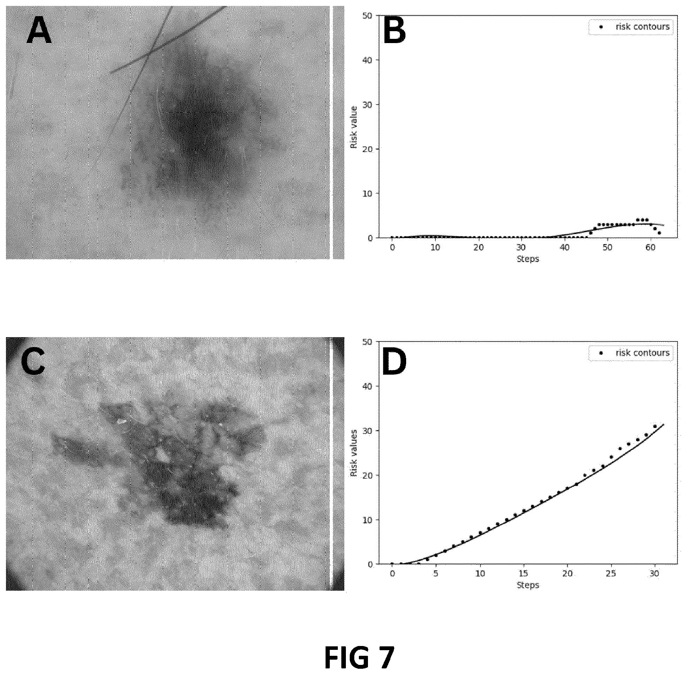

The number of contours with abnormal color distribution ("risky contours") per repetition was plotted against the number of repetitions as shown in Figure 7. From the output graph the following metrics are extracted:

$$\frac{\widehat{r_n}}{k} = 0.085 \text{ for the nevus (Figures 7A, 7B),}$$

and

$$\frac{\widehat{r_n}}{k} = 1 \text{ for the melanoma (Figures 7C, 7D).}$$

Example 2

Analysis of digital images for the detection of malignant or benign skin lesions.

Using the image analysis method disclosed herein, the inventors analyzed 600 digital images of skin lesions in order to determine whether the lesion is a melanoma or nevus. The methodological steps were incorporated into a computer algorithm which analyzed every image independently. Following the calibration performed in Example 1, the classification threshold $R_c$ was set at 0.232.

The results showed that the detection of color abnormalities was correlated to melanoma conditions. Color abnormalities were found in 96% of the pictures of a melanoma whereas they were detected in only 10% of non-melanoma pictures. This corresponds to a 93% accuracy for the methodology which is greater than the accuracy normally achieved by the trained eye of a medical expert which rarely exceeds 70%, mainly due to low specificity levels when no preliminary diagnosis occurs.

**Claims**

1. A computer implemented method of detecting melanoma in a digital skin image, comprising the following steps:

   a) determining (101, 201) the area of interest AOI, in the digital image through an image segmentation algorithm, masking the AOI from the outside area, and converting the masked AOI and the area outside the AOI into grayscale;
   b) calculating (102, 202) the mean grayscale intensity value and the standard deviation for the AOI and for the area outside the AOI to extract a risk threshold as a function of the mean and standard deviation;
   c) dividing (104, 206) the color of the AOI into contours by applying a grayscale intensity threshold value to the image;
   d) if multiple contours are identified in step c), measuring the mass center of all contours identified and calculating (105, 207) the euclidean distance of each contour center from said mass center of all contours;
   e) for each contour identified in step c), calculating (106, 208) the ratio of the average plus the standard deviation of all the measured distances divided by each contour's distance from the mass center of all contours measured in step d);

f) comparing (107, 209) said ratio with the risk threshold extracted in step b) whereby, if said ratio is higher than the risk threshold extracted in step b), then said contour is classified as having abnormal color distribution; and

g) repeating steps c) to f) by applying multiple threshold values to the image, until the size of a contour covers the AOI;

wherein the presence of multiple contours having abnormal color distribution and whose numbers are increasing during the process is an indication of a melanoma.

2. The method according to claim 1, wherein if a sub-contour, i.e. a contour inside a contour, is detected, then step c) further comprises measuring the average color intensity of an area extending from 5 to 15 pixels towards all directions around the mass center of said contour, measuring the average color intensity of an area extending from 5 to 15 pixels towards all directions around the mass center of said sub-contour; wherein if the difference of the mean values of the two areas is higher than 30 then said sub-contour is classified as having abnormal color distribution.

3. The method according to claims 1 or 2, further comprises calculating a risk value during each repetition, and wherein said risk value increases every time a contour and/or sub-contour is classified as having abnormal color distribution; whereby the cumulative risk value (211) obtained at the end of the process is used to classify (212) the AOI as a melanoma or as a non-melanotic lesion.

4. The method according to claim 3, wherein the ratio of the cumulative risk value obtained at the end of the process divided by the total number of repetitions is compared to a classification threshold value ranging from 0.18 to 0.25, whereby if said ratio is greater than the classification threshold value it is an indication of a melanoma.

5. The method according to any one of the previous claims, wherein step b) further comprises calculating (203) the symmetry of the AOI, wherein an asymmetric AOI is an indication of a melanoma.

6. The method according to any one of the previous claims, wherein step b) further comprises determining (204) whether the borders of the AOI are anomalous, by calculating the geometric center of the AOI; calculating the euclidean distance of each pixel at the border of the AOI from said geometric center; normalizing the calculated distances by subtracting their average value; and identifying the presence of a peak each time the normalized distance changes sign between two consecutive points;

wherein the border of the AOI is anomalous when the number of peaks is more than five;
and wherein an anomalous border of the AOI is an indication of a melanoma.

7. An apparatus for detecting a melanoma in a digital image of a tissue, the apparatus comprising: a camera adapted to obtain said image; a processor adapted to process the image data according to the method of any of the preceding claims; and an output device adapted to indicate a likelihood of the presence or absence of a melanoma.

8. The apparatus according to claim 7, wherein said apparatus is a smartphone or a tablet.

9. A computer program comprising instructions which, when the program is executed by a computer, cause the computer to carry out the method according to claims 1 to 6.

**Patentansprüche**

1. Ein computerimplementiertes Verfahren zur Detektion eines Melanoms in einem digitalen Hautbild, umfassend die folgenden Schritte:

a) Bestimmen (101, 201) des Bereichs von Interesse, AOI, in dem digitalen Bild mittels eines Bildsegmentierungsalgorithmus, Maskieren der AOI gegenüber dem Außenbereich sowie Umwandeln der maskierten AOI und des Bereichs außerhalb der AOI in Graustufen;

b) Berechnen (102, 202) des mittleren Grauwertintensitätswerts und der Standardabweichung für die AOI sowie für den Bereich außerhalb der AOI zur Extraktion eines Risikoschwellenwerts als Funktion des Mittelwerts und der Standardabweichung;

c) Unterteilen (104, 206) der Farbe der AOI in Konturen durch Anwenden eines Grauwert-Intensitätsschwellenwerts auf das Bild;

d) sofern in Schritt c) mehrere Konturen identifiziert werden, Messen des Massenzentrums aller identifizierten Konturen und Berechnen (105, 207) der euklidischen Distanz jedes Konturenzentrums zu dem genannten Massenzentrum aller Konturen;

e) für jede in Schritt c) identifizierte Kontur, Berechnen (106, 208) des Verhältnisses aus der Summe von Mittelwert und Standardabweichung aller gemessenen Distanzen, dividiert durch den in Schritt d) gemessenen Distanz der jeweiligen Kontur zum Massenzentrum aller Konturen;

f) Vergleichen (107, 209) des genannten Verhältnisses mit dem in Schritt b) extrahierten Risikoschwellenwert, wobei, wenn das genannte Verhältnis höher ist als der in Schritt b) extrahierte Risikoschwellenwert, die betreffende Kontur als eine Kontur mit anormaler Farbverteilung klassifiziert wird; und

g) Wiederholen der Schritte c) bis f) durch Anwenden mehrerer Schwellenwerte auf das Bild, bis die Größe einer Kontur die AOI abdeckt;

wobei das Vorhandensein mehrerer Konturen mit anormaler Farbverteilung, deren Anzahl im Verlauf des Verfahrens zunimmt, ein Hinweis auf ein Melanom ist.

2. Verfahren nach Anspruch 1, wobei, wenn eine Subkontur, d. h. eine innerhalb einer Kontur liegende Kontur, detektiert wird, Schritt c) ferner umfasst: Messen der durchschnittlichen Farbintensität eines Bereichs, der sich von 5 bis 15 Pixeln in alle Richtungen um das Massenzentrum der genannten Kontur erstreckt, sowie Messen der durchschnittlichen Farbintensität eines Bereichs, der sich von 5 bis 15 Pixeln in alle Richtungen um das Massenzentrum der genannten Subkontur erstreckt; wobei, wenn die Differenz der Mittelwerte der beiden Bereiche größer als 30 ist, die genannte Subkontur als eine Kontur mit anormaler Farbverteilung klassifiziert wird.

3. Verfahren nach einem der Ansprüche 1 oder 2, ferner umfassend das Berechnen eines Risikowerts bei jeder Wiederholung, wobei sich der genannte Risikowert jedes Mal erhöht, wenn eine Kontur und/oder Subkontur als eine Kontur mit anormaler Farbverteilung klassifiziert wird; wobei der am Ende des Verfahrens erhaltene kumulative Risikowert (211) zur Klassifizierung (212) der AOI als Melanom oder als nicht-melanotische Hautläsion verwendet wird.

4. Verfahren nach Anspruch 3, wobei das Verhältnis des am Ende des Verfahrens erhaltenen kumulativen Risikowerts geteilt durch die Gesamtanzahl der Wiederholungen mit einem Klassifikationsschwellenwert im Bereich von 0,18 bis 0,25 verglichen wird, wobei, wenn das genannte Verhältnis größer ist als der Klassifikationsschwellenwert, dies ein Hinweis auf ein Melanom ist.

5. Verfahren nach einem der vorhergehenden Ansprüche, wobei Schritt b) ferner umfasst das Berechnen (203) der Symmetrie der AOI, wobei eine asymmetrische AOI ein Hinweis auf ein Melanom ist.

6. Verfahren nach einem der vorhergehenden Ansprüche, wobei Schritt b) ferner umfasst das Bestimmen (204), ob die Ränder der AOI anomal sind, durch: Berechnen des geometrischen Zentrums der AOI; Berechnen der euklidischen Distanz jedes Pixels am Rand der AOI zu dem genannten geometrischen Zentrum; Normalisieren der berechneten Distanzen durch Subtrahieren ihres Mittelwerts; und Identifizieren des Auftretens einer Spitze jedes Mal, wenn die normalisierte Distanz zwischen zwei aufeinanderfolgenden Punkten ihr Vorzeichen ändert;

wobei der Rand der AOI als anomal angesehen wird, wenn die Anzahl der Spitzen größer als fünf ist; und wobei ein anomaler Rand der AOI ein Hinweis auf ein Melanom ist.

7. Vorrichtung zur Detektion eines Melanoms in einem digitalen Bild eines Gewebes, wobei die Vorrichtung umfasst: eine Kamera, die dazu ausgelegt ist, das genannte Bild aufzunehmen; einen Prozessor, der dazu ausgelegt ist, die Bilddaten gemäß dem Verfahren nach einem der vorhergehenden Ansprüche zu verarbeiten; und eine Ausgabeeinrichtung, die dazu ausgelegt ist, eine Wahrscheinlichkeit für das Vorhandensein oder Nichtvorhandensein eines Melanoms anzuzeigen.

8. Vorrichtung nach Anspruch 7, wobei die genannte Vorrichtung ein Smartphone oder ein Tablet ist.

9. Computerprogramm, umfassend Anweisungen, die, wenn das Programm von einem Computer ausgeführt wird, den Computer veranlassen, das Verfahren nach den Ansprüchen 1 bis 6 durchzuführen.

**Revendications**

1. Procédé mis en œuvre par ordinateur de détection d'un mélanome dans une image cutanée numérique, comprenant les étapes suivantes:

   a) déterminer (101, 201) la zone d'intérêt, AOI, dans l'image numérique au moyen d'un algorithme de segmentation d'image, masquer l'AOI par rapport à la zone extérieure, et convertir l'AOI masquée et la zone extérieure à l'AOI en niveaux de gris;
   b) calculer (102, 202) la valeur moyenne d'intensité en niveaux de gris et l'écart type pour l'AOI et pour la zone extérieure à l'AOI afin d'extraire un seuil de risque en fonction de la moyenne et de l'écart type;
   c) diviser (104, 206) la couleur de l'AOI en contours en appliquant une valeur seuil d'intensité en niveaux de gris à l'image;
   d) si plusieurs contours sont identifiés à l'étape c), mesurer le centre de masse de tous les contours identifiés et calculer (105, 207) la distance euclidienne entre le centre de chaque contour et ledit centre de masse de tous les contours;
   e) pour chaque contour identifié à l'étape c), calculer (106, 208) le rapport de la moyenne plus l'écart type de toutes les distances mesurées divisé par la distance de chaque contour par rapport au centre de masse de tous les contours mesurée à l'étape d);
   f) comparer (107, 209) ledit rapport avec le seuil de risque extrait à l'étape b), de telle sorte que, si ledit rapport est supérieur au seuil de risque extrait à l'étape b), ledit contour est classé comme présentant une distribution anormale des couleurs ; et
   g) répéter les étapes c) à f) en appliquant plusieurs valeurs seuils à l'image, jusqu'à ce que la taille d'un contour couvre l'AOI;

   dans lequel la présence de plusieurs contours présentant une distribution anormale des couleurs et dont le nombre augmente au cours du processus constitue une indication d'un mélanome.

2. Procédé selon la revendication 1, dans lequel, si un sous-contour, c'est-à-dire un contour à l'intérieur d'un contour, est détecté, l'étape c) comprend en outre la mesure de l'intensité moyenne des couleurs d'une zone s'étendant de 5 à 15 pixels dans toutes les directions autour du centre de masse dudit contour, la mesure de l'intensité moyenne des couleurs d'une zone s'étendant de 5 à 15 pixels dans toutes les directions autour du centre de masse dudit sous-contour; dans lequel, si la différence des valeurs moyennes des deux zones est supérieure à 30, ledit sous-contour est classé comme présentant une distribution anormale des couleurs.

3. Procédé selon la revendication 1 ou 2, comprenant en outre le calcul d'une valeur de risque à chaque répétition, et dans lequel ladite valeur de risque augmente chaque fois qu'un contour et/ou un sous-contour est classé comme présentant une distribution anormale des couleurs; de telle sorte que la valeur de risque cumulée (211) obtenue à la fin du processus est utilisée pour classer (212) l'AOI comme mélanome ou comme lésion non mélanotique.

4. Procédé selon la revendication 3, dans lequel le rapport de la valeur de risque cumulée obtenue à la fin du processus divisé par le nombre total de répétitions est comparé à une valeur seuil de classification comprise entre 0,18 et 0,25, de telle sorte que, si ledit rapport est supérieur à la valeur seuil de classification, cela constitue une indication d'un mélanome.

5. Procédé selon l'une quelconque des revendications précédentes, dans lequel l'étape b) comprend en outre le calcul (203) de la symétrie de l'AOI, dans lequel une AOI asymétrique constitue une indication d'un mélanome.

6. Procédé selon l'une quelconque des revendications précédentes, dans lequel l'étape b) comprend en outre la détermination (204) du caractère anormal des bordures de l'AOI, en calculant le centre géométrique de l'AOI; en calculant la distance euclidienne de chaque pixel à la bordure de l'AOI par rapport audit centre géométrique; en normalisant les distances calculées en soustrayant leur valeur moyenne; et en identifiant la présence d'un pic chaque fois que la distance normalisée change de signe entre deux points consécutifs;

   dans lequel la bordure de l'AOI est anormale lorsque le nombre de pics est supérieur à cinq;
   et dans lequel une bordure anormale de l'AOI constitue une indication d'un mélanome.

7. Appareil de détection d'un mélanome dans une image numérique d'un tissu, l'appareil comprenant: une caméra adaptée pour obtenir ladite image; un processeur adapté pour traiter les données d'image selon le procédé de l'une quelconque des revendications précédentes; et un dispositif de sortie adapté pour indiquer une probabilité de présence ou d'absence d'un mélanome.

8. Appareil selon la revendication 7, dans lequel ledit appareil est un smartphone ou une tablette.

9. Programme d'ordinateur comprenant des instructions qui, lorsque le programme est exécuté par un ordinateur, amènent l'ordinateur à mettre en œuvre le procédé selon les revendications 1 à 6.

Detection of AOI through an image
segmentation algorithm `101`

`100`

Calculation of mean value and standard
deviation for the AOI and for the outside area
=> extraction of risk threshold `102`

Calculation of the geometric center of the AOI `103`

Division of the color of the AOI into contours `104`

For each contour: Calculation of euclidean
distance of the mass center `105`

Multiple
repetitions

For each contour: Calculation of the ratio of
the average plus the standard deviation of
all measured distances divided by each
contour distance `106`

For each contour: Comparison of ratio to risk
threshold => contour classified as having
normal or abnormal color distribution `107`

Calculation of the number of contours having
abnormal color distribution identified during
each repetition `108`

# FIG 1

Detection of AOI and outside area (OA) Masking & conversion into greyscale — 201

Calculation of geometric center of the AOI Calculation of mean & standard deviation for the AOI & OA => extraction of risk threshold — 202

— 200

Calculation of symmetry of the AOI — 203

Measurement of noise at the borders of the AOI & of number of peaks around borders — 204

Application of bilateral filter to smoothen noise — 205

Division of color of the AOI into contours — 206

For each contour: Calculation of euclidean distance of the mass center — 207

For each contour: Calculation of the ratio of the average plus the standard deviation of all measured distances divided by each contour distance — 208

For each contour: Comparison of ratio to risk threshold => contour classified as having normal or abnormal color distribution — 209

Comparison of average color intensity of an area around the center of each sub-contour & of the corresponding contour => sub-contour classified as having normal or abnormal color distribution — 210

Multiple repetitions

Cumulative risk value calculated — 211

Comparison to classification threshold — 212

# FIG 2

**FIG 3**

FIG 4

**FIG 5**

FIG 6

**FIG 6 (cont.)**

**FIG 7**

**EP 4 528 634 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DO T-T et al.** *IEEE Transactions on Multimedia*, 2018, vol. 20 (10), 2849-2864
- **MAHDIRAJI SA et al.** *2017 3rd International Conference on Pattern Recognition and Image Analysis (IPRIA)*, 2017, 102-107
- **ABDER-RAHMAN HA et al.** *IEEE Access*, 2020, vol. 8, 83333-83346